(19) Europäisches Patentamt
European Patent Office
Office européen des brevets

(11) Numéro de publication: **0 184 957**
**B1**

(12) # FASCICULE DE BREVET EUROPÉEN

(45) Date de publication du fascicule du brevet: **19.04.89**

(51) Int. Cl.⁴: **C 07 D 405/04**, C 07 D 409/04, A 61 K 31/44

(21) Numéro de dépôt: **85402236.5**

(22) Date de dépôt: **19.11.85**

(54) **-N-méthyl(méthyl-6 pyridyl-2)-2 dithianne-1,3 carbothioamide-2, sa préparation et les médicaments qui la contiennent.**

(30) Priorité: **20.11.84 FR 8417648**

(43) Date de publication de la demande: **18.06.86 Bulletin 86/25**

(45) Mention de la délivrance du brevet: **19.04.89 Bulletin 89/16**

(84) Etats contractants désignés: **AT BE CH DE FR GB IT LI LU NL SE**

(56) Documents cité:
**EP-A-0 046 417**
**EP-A-0 073 704**
**EP-A-0 097 584**
**GB-A-2 046 265**

**"Medicinal Chemistry, 3rd. ed., part I, A. Burger", p. 75**

**Le dossier contient des informations techniques présentées postérieurement au dépôt de la demande et ne figurant pas dans le présent fascicule.**

(73) Titulaire: **RHONE- POULENC SANTE, 20, avenue Raymond Aron, F-92160 Antony (FR)**

(72) Inventeur: **Aloup, Jean- Claude, 53, rue Marcel Risser, F-94290 Villeneuve- le- Roi (FR)**
Inventeur: **Bouchaudon, Jean, 23, avenue des Saules, F-91390 Morsang- sur- Orge (FR)**
Inventeur: **Farge, Daniel, 30, rue des Pins Sylvestres, F-94320 Thiais (FR)**
Inventeur: **James, Claude, 31 bis, avenue Gambetta, F-75020 Paris (FR)**

(74) Mandataire: **Le Goff, Yves, RHONE- POULENC RECHERCHES Brevets Pharma 25, Quai Paul Doumer, F-92408 Courbevoie (FR)**

EP 0 184 957 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne un nouveau dérivé de la thioformamide de formule:

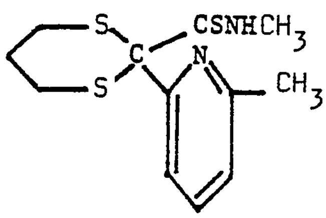

(I)

Selon l'invention, le produit de formule (I) peut être préparé par action de la méthilamine sur un dithioester de formule générale:

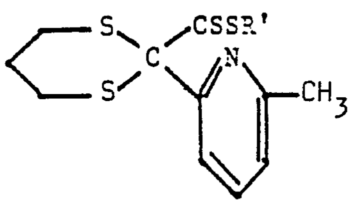

(II)

dans laquelle R' représente un radical alcoyle contenant 1 à 4 atomes de carbone en chaîne droite ou ramifiée ou un radical benzyle ou carboxyméthyle.

Généralement, on opère avec un excès d'amine sans solvant ou dans un solvant organique tel qu'un carbure aromatique, un éther ou un alcool à bas poids moléculaire ou un mélange de ces solvants, à une température comprise entre 20 et 130° C, éventuellement sous pression.

Les dithioesters de formule générale (II) peuvent être obtenus par action d'un dérivé organo-lithien sur un produit de formule:

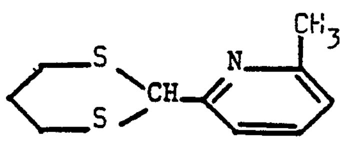

(III)

suivie de l'action de sulfure de carbone puis d'un produit de formule générale:

R'-Z (IV)

dans laquelle R' est défini comme précédemment et Z représente un atome d'halogène, de préférence un atome de chlore, de brome, ou d'iode ou un reste d'ester réactif, de préférence un reste mésyloxy ou tosyloxy.

La réaction s'effectue généralement dans un solvant organique anhydre tel que l'hexaméthylphosphorotriamide, additionné généralement d'un éther tel que le tétrahydrofuranne, à une température comprise entre -80 et -40° C.

Les dérivés organo-lithiens qui conviennent particulièrement bien sont de préférence les alcoylures de lithium tels que le butyllithium et l'isopropyllithium ou le phényllithium, en solution dans un solvant inerte comme l'hexane, ou les amidures de lithium tels que le diisopropylamidure de lithium ou l'isopropylamidure de lithium.

Les produits de formule (III) peuvent être préparés par action de propanedithiol sur la méthyl-6 pyridinecarboxoldéhyde-2 en opérant dans un solvant permettant d'éliminer par distillation azéotropique l'eau formée en cours de réaction. Dans la pratique, on utilise de préférence le benzène, le toluène, les xylènes ou le dichloro-1,2 éthane.

Le nouveau produit selon la présente invention peut être purifié par les méthodes physiques habituelles, notamment la cristallisation et la chromatographie.

On connaît par les demandes de brevets européen n° 0 046 417 et français n° 7 908 032 ou son équivalent GB-2 046 265 des thioformamides possédant des propriétés antisécrétoires.

Le nouveau produit selon l'invention présente également des propriétés antisécrétoires qui peuvent être mises en évidence chez le rat par voie orale, en particulier dans la technique de ROSSI et coll. C.R. Soc. Biol., 150, 2124 (1956) et celle de SHAY et coll., Gastroenterology, 5, 43 (1945). Sa dose létale ($DL_{50}$) chez la souris est supérieure à 900 mg/kg par voie orale.

Le produit selon l'invention présente en outre, et c'est ce qui fait son originalité, des avantages importants que ne possèdent pas les produits de l'art antérieur. Il présente en effet une plus grande durée d'action et/ou

une plus faible toxicité.

L'exemple suivant, donné à titre non limitatif, montre comment l'invention peut être mise en pratique.

**Exemple 1**

A une suspension de 130,5 g de (méthyl-6 pyridyl-2)-2 dithianne-1,3 carbodithioate-2 de méthyle dans 1,2 litre d'éthanol maintenue à une température voisine de 20°C, on ajoute goutte à goutte en 10 minutes 840 $cm^3$ d'une solution à 33 % (poids/volume) de méthylamine dans l'éthanol. Le mélange est ensuite agité pendant 4 heures à la même température. Les cristaux apparus sont séparés par filtration, lavés 2 fois par 100 $cm^3$ au total d'acétate d'éthyle, 4 fois par 400 $cm^3$ au total d'oxyde d'isopropyle et séchés sous pression réduite (0,2 mm de mercure; 0,03 kPa) à une température voisine de 20°C. Le produit ainsi obtenu (100,2 g), additionné de 18,4 g de produit préparé dans les mêmes conditions dans une opération antérieure, est dissous dans 3,3 litres d'acétate d'éthyle bouillant; la solution est additionnée de 33 g de noir décolorant et filtrée à chaud. Le filtre est lavé 4 fois par 400 $cm^3$ au total d'acétate d'éthyle bouillant. Le filtrat et les liqueurs de lavage sont réunis et refroidis pendant 4 heures à une température voisine de 0°C. Les cristaux apparus sont séparés par filtration, lavés 4 fois par 200 $cm^3$ au total d'acétate d'éthyle, 6 fois par 600 $cm^3$ au total d'oxyde d'isopropyle, puis séchés sous pression réduite (0,2 mmHg; 0,03 kPa) à 55°C. On obtient ainsi 99,1 g de N-méthyl (méthyl-6 pyridyl-2)-2 dithianne-1,3 carbothioamide-2 fondant à 168°C.

Le (méthyl-6 pyridyl-2)-2 dithianne-1,3 carbodithioate-2 de méthyle peut être préparé de la façon suivante:

A 440 $cm^3$ d'une solution 1,6 M de n-butyllithium dans l'hexane, maintenue sous atmosphère d'argon et refroidie à -71°C, on ajoute goutte à goutte en 20 minutes 440 $cm^3$ d'un mélange d'hexaméthylphosphorotriamide et de tétrahydrofuranne anhydres (47 - 53 en volumes). On ajoute ensuite en 30 minutes et à une température voiaine de -70°C une solution de 97 g de (méthyl-6 pyridyl-2)-2 dithianne-1,3 dans 300 $cm^3$ d'un mélange d'hexaméthylphosphorotriamide et de tétrahydrofuranne anhydres (47 - 53 en volumes). Après 1 heure 40 minutes d'agitation, on ajoute en 40 minutes 53 g de sulfure de carbone en solution dans 220 $cm^3$ de tétrahydrofuranne anhydre. Après 45 minutes d'agitation à -70°C, on ajoute en 30 minutes et à la même température 97 g d'iodure de méthyle en solution dana 220 $cm^3$ de tétrahydrofuranne anhydre. Le mélange réactionnel est ensuite agité pendant 30 minutes à une température voisine de -65°C puis pendant 50 minutes en laissant remonter progresaivement la température à 10°C; il est ensuite versé sur un mélange de 600 $cm^3$ d'eau distillée et de 500 $cm^3$ d'acétate d'éthyle. Après décantation, la phase aqueuse est extraite 2 fois par 600 $cm^3$ au total d'acétate d'éthyle. Les extraits organiques sont réunis, lavés 2 fois par 600 $cm^3$ au total d'une solution aqueuse saturée de chlorure de sodium, additionnés de sulfate de magnésium anhydre et de noir décolorant, conservés pendant 15 heures à une température voisine de 5°C, filtrés puis concentrés sous pression réduite (25 mm de mercure; 3,25 kPa) à 50°C. Le produit obtenu (192,2 g) est additionné de 231,7 g de produit préparé dans les mêmes conditions dans une opération antérieure, et est chromatographié sur 1,5 kg de gel de silice neutre (0,063 - 0,200 mm) contenus dans une colonne de 8 cm de diamètre. On élue par du chlorure de méthylène en recueillant une fraction de 3,5 litres puis 7 fractions de 600 $cm^3$. Les fractions 5 à 8 sont réunies et concentrées à sec sous pression réduite (25 mm de mercure; 3,25 kPa) à 50°C. Le produit obtenu (276,5 g) est dissous dans 200 $cm^3$ d'acétate d'éthyle bouillant. La solution obtenue est refroidie et conservée pendant 1 heure à une température voisine de 5°C. Les cristaux apparus sont séparés par filtration, lavés 2 fois par 60 $cm^3$ au total d'acétate d'éthyle puis 3 fois par 135 $cm^3$ au total d'oxyde d'isopropyle et séchés sous pression réduite (0,2 mm de mercure; 0,03 kPa) à 20°C. On obtient ainsi 153,9 g de (méthyl-6 pyridyl-2)-2 dithiane-1,3 carbodithioate-2 de méthyle fondant à 92°C.

Le (méthyl-6 pyridyl-2)-2 dithianne-1,3 peut être préparé de la manière suivante:

Une solution de 100 g de méthyl-6 pyridinecarboxaldéhyde-2, de 285 g de propanedithiol-1,3 et de 11,8 g d'acide paratoluènesulfonique, dans 2,6 litres de toluène est chauffée à l'ébullition pendant 12 heures et 40 minutes en éliminant par azéotropie l'eau formée. Après refroidissement à une température voisine de 20°C, le mélange réactionnel est versé sur un mélange de 1 litre d'eau distillée et de 500 g de glace puis additionné goutte à goutte d'une solution aqueuse environ 10 N de potasse. Après décantation, la solution organique est lavée par une solution aqueuse environ 5N de potasse, 3 fois par 1700 $cm^3$ au total d'eau distillée, séchée sur du sulfate de magnésium anhydre et concentrée à sec sous pression réduite (25 mm de mercure; 3,25 kPa) à 50°C. Le produit obtenu (160,2 g) est chromatographié sur 980 g de gel de silice neutre (0, 063 - 0,200 mm) contenus dans une colonne de 8 cm de diamètre. On élue par un mélange de cyclohexane et d'acétate d'éthyle (70 - 30 en volumes) en recueillant des fractions de 300 $cm^3$. Les fractions 9 à 23 sont réunies et concentrées à sec sous pression réduite (25 mm de mercure; 3,25 kPa) à 50°C. On obtient ainsi 90,2 g de (méthyl-6 pyridyl-2)-2 dithiane-1,3 fondant à 74°C.

La présente invention concerne également les médicaments constitués par le produit de formule (I), à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose,

lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositiona peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent, outre le produit actif, des excipients tels que le beurre de cacao, des glycérides semi-synthétiques ou des polyéthylèneglycols.

En thérapeutique humaine le produit de l'invention est particulièrement utile dans le traitement des ulcères gastro-intestinaux. Les doses dépendent de l'effet recherché et de la durée du traitement; elles sont généralement comprises entre 25 et 1000 mg par jour par voie orale pour un adulte en une ou plusieurs prises.

D'une façon générale, le médecin déterminera la posologie qu'il estime la plus appropriée en fonctin de l'âge, du poids et de tous les autres facteurs propres au sujet à traiter.

L'exemple suivant, donné à titre non limitatif, illustre une composition selon l'invention.

**Exemple A**

On prépare, selon la technique habituelle, des comprimés dosés à 50 mg de produit actif ayant la composition suivante:

| | |
|---|---|
| - N-méthyl (méthyl-6 pyridyl-2)-2 dithianne-1,3 carbothioamide-2 | 50 mg |
| - amidon | 60 mg |
| - lactose | 50 mg |
| - stéarate de magnésium | 2 mg |

**Revendications** pour les états contractants: BE,CH, DE, DE, GB, IT, LI, LU, NL, SE

1. Un nouveau dérivé de la thioformamide, caractérisé en ce qu'il répond à la formule:

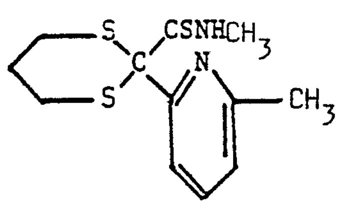

(I)

2. Procédé de préparation du produit selon la revendication 1, caractérisé en ce que l'on fait réagir la méthylamine sur un dithioester de formule générale:

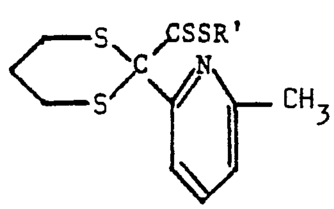

(II )

dans laquelle R' représente un radical alcoyle contenant 1 à 4 atomes de carbone en chaîne droite ou ramifiée ou un radical benzyle ou carboxyméthyle, puis isole le produit obtenu.

3. Médicament caractérisé en ce qu'il consiste en le produit selon la revendication 1.

4. Composition pharmaceutique caractérisée en ce qu'elle contient à titre de produit actif le produit selon la revendication 1 éventuellement en association avec un ou plusieurs diluants ou adjuvants compatibles et pharmaceutiquement acceptables.

**Revendication** pour l'état contractant: AT

1. Procédé de préparation d'un nouveau dérivé de la thioformamide, de formule:

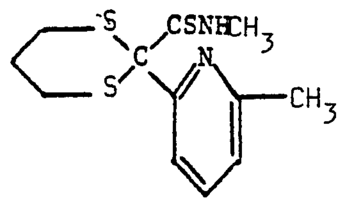

(I)

caractérisé en ce que l'on fait réagir la méthylamine sur un dithioester de formule générale:

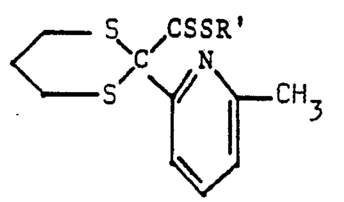

(II )

dans laquelle le symbole R' représente un radical alcoyle contenant 1 à 4 atomes de carbone en chaîne droite ou ramifiée ou un radical benzyle ou carboxyméthyle, puis isole le produit obtenu.

**Patentansprüche** für die Vertragsstaaten: BE, CH, DE, FR, GB, IT, LI, LU, NL, SE

1. Ein neues Derivat des Thioformamids, dadurch gekennzeichnet, daß es der Formel:

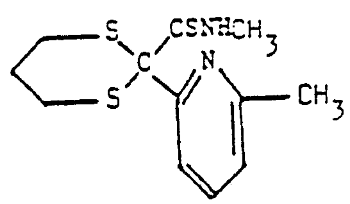

(I)

entspricht.

2. Verfahren zur Herstellung des Produkts gemäß Anspruch 1, dadurch gekennzeichnet, daß man Methylamin auf einen Dithioester der allgemeinen Formel:

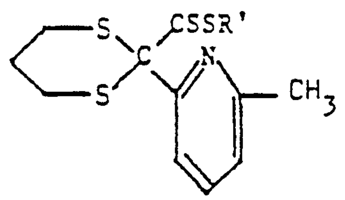

(II)

worin R' einen Alkylrest mit 1 bis 4 Kohlenstoffatomen in gerader oder verzweigter Kette oder einen Benzyl- oder Carboxymethylrest bedeutet, einwirken läßt, und dann das erhaltene Produkt isoliert.

3. Arzneimittel, dadurch gekennzeichnet, daß es aus dem Produkt gemäß Anspruch 1 besteht.

4. Pharmazeutische Zusammensetzung, dadurch gekennzeichnet, daß sie als Wirkstoff das Produkt gemäß Anspruch 1 enthalt, gegebenenfalls zusammen mit einem oder mehreren verträglichen und pharmazeutisch annehmbaren Verdünnungs- oder Hilfsmitteln.

**Patentanspruch** für den Vertragsstaat: AT

1. Verfahren zur Herstellung eines neuen Thioformamidderivets der Formel:

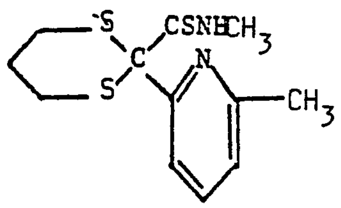

(I)

dadurch gekennzeichnet, dass man Methylamin mit einem Dithioester der allgemeinen Formel:

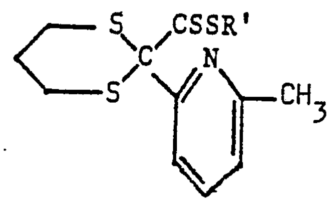

(II)

in welcher das Symbol R' einen Alkylrest mit 1 bis 4 Kohlenstoffatomen in gerader oder verzweigter Kette oder einen Benzyl- oder carboxymethylrest darstellt, reagieren lässt und dann das erhaltene Produkt isoliert.

**Claims** for the contracting states: BE CH, DE, FR, GB, IT, LI, LU, NL, SE

1. A new thioformamide derivative which is of the formula:

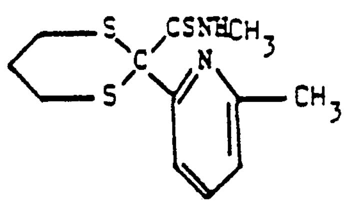

(I)

2. A process for preparing the product according to claim 1, wherein methylamine is reacted with a dithioester of general formula:

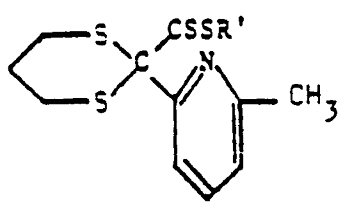

(II)

in which R′ denotes a straight-chain or branched alkyl radical containing 1 to 4 carbon atoms or a benzyl or carboxymethyl radical, and the product obtained is then isolated.

3. A drug which consists of the product according to claim 1.

4. A pharmaceutical composition which contains by way of active product the product according to claim 1, optionally in combination with one or more diluents or adjuvants which are compatible and pharmaceutically acceptable.

**Claim** for the contracting state: AT

1. A process for preparing a new thioformamide derivative of formula:

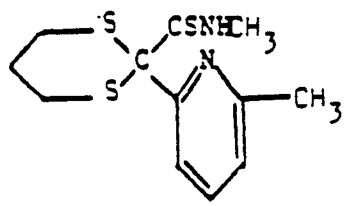

(I)

wherein methylamine is reacted with a dithioester of general formula:

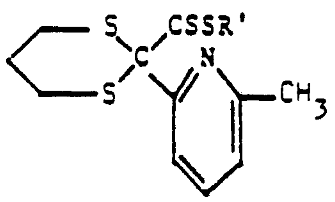

(II)

in which the symbol R′ denotes a straight-chain or branched alkyl radical containing 1 to 4 carbon atoms or a benzyl or carboxymethyl radical, and the product obtained is then isolated.